# FASCICULE DE BREVET EUROPEEN

(11) **EP 2 374 449 B1**
(45) Date de publication et mention de la délivrance du brevet: **13.01.2016**
(21) Numéro de dépôt: 11173086.7
(22) Date de dépôt: 18.04.2007
(51) Int. Cl.: A61K 9/107, A61K 31/7048, A61P 17/00, A61P 17/08, A61P 17/10, A61K 47/14, A61K 47/44, A61K 47/10, A61K 47/32, A61K 47/34

(54) **Composition comprenant au moins une phase aqueuse et au moins une phase grasse comprenant de l'ivermectine**
Zusammensetzung, die aus mindestens einer wässrigen Phase und mindestens einer Ivermectin enthaltenden Fettphase besteht
Composition including at least one aqueous phase and at least one oil phase including ivermectin

(30) Priorité: 19.04.2006 FR 0603452
(43) Date de publication de la demande: 12.10.2011
(62) Demande divisionnaire de: 07731904.4
(73) Titulaire: Galderma S.A., 6330 Cham (CH)
(72) Inventeur: Diaz-Astruc, Fanny, Houston, TX 77077 (US); Barthez, Nathalie, 06700 Saint Laurent du Var (FR); Segura, Sandrine, 06410 Biot (FR)
(74) Mandataire: Casalonga

(56) Documents cités:
- WO-A-00/42990
- WO-A-00/74489
- WO-A-02/09764
- WO-A-2004/093886
- WO-A-2005/089806
- WO-A1-2006/069580
- GB-A- 2 310 801

## Description

La présente invention se rapporte à une composition pharmaceutique à base d'un composé de la famille des avermectines comprenant au moins une phase grasse et au moins une phase aqueuse, ledit composé de la famille des avermectines étant solubilisé dans ladite phase grasse.
La présente invention se rapporte plus particulièrement à une composition pharmaceutique à base d'ivermectine comprenant au moins une phase grasse et au moins une phase aqueuse, l'ivermectine étant solubilisée dans ladite phase grasse.

Elle porte également sur son procédé de préparation et son utilisation dans la fabrication d'une préparation pharmaceutique destinée au traitement des affections dermatologiques, en particulier de la rosacée.

L'ivermectine est un mélange de deux composés appartenant à la classe des avermectines, la 5-0-demethyl-22,23-dihydroavermectine A₁ₐ et la 5-0-demethyl-22,23-dihydroavermectine A_{1b}. Ils sont également connus sous le nom de 22,23-dihydroavermectine B₁ₐ et 22,23-dihydroavermectine B_{1b}. L'ivermectine contient au moins 80% de 22,23-dihydroavermectine B₁ₐ et moins de 20% de 22,23-dihydroavermectine B_{1b}. Cet agent actif fait partie de la classe des avermectines, un groupe de lactones macrocycliques produit par le bacterium *Streptomyces avermitilis* (Reynolds JEF (Ed) (1993) Martindale. The extra pharmacopoeia. 29th Edition. Pharmaceutical Press, London). Les avermectines incluent notamment l'ivermectine, l'invermectine, l'avermectine, l'abamectine, la doramectine, l'eprinomectine et la sélamectine.

L'ivermectine est plus particulièrement un antihelminthique. Il est déjà décrit chez l'homme dans le traitement de l'onchocercose à Onchocerca volvulus, de la strongyloïdose (anguillulose) gastro-intestinale (produit Stromectol®), de la gale sarcoptique humaine (Meinking TL et al., N Engl J Med 1995 Jul 6;333(1):26-30 The treatment of scabies with ivermectin) ainsi que dans le traitement de la microfilarémie diagnostiquée ou suspectée chez les sujets atteints de la filariose lymphatique due à Wuchereria bancrofti.

Les affections dermatologiques sont souvent liées à une sensibilité accrue de la peau, particulièrement dans le cas de la rosacée qui est une dermatose inflammatoire qui affecte principalement la partie centrale du visage et se caractérise entre autres par le rougissement du visage, des bouffées de chaleur, un érythème facial. Ce type de pathologie nécessite particulièrement l'utilisation de formulations galéniques permettant un étalement facile et donnant à l'utilisateur une sensation agréable et de bien-être.

Il existe donc un besoin de disposer d'une composition pharmaceutique topique contenant au moins un composé de la famille des avermectines, et plus particulièrement de l'ivermectine qui soit parfaitement adaptée à la pathologie et spécifiquement aux peaux sensibilisées, qui soit industriellement acceptable, c'est-à-dire dont la formulation soit stable physiquement (sans séparation de phase), et stable chimiquement (sans modification de la stabilité de l'actif) et qui optimise la pénétration de l'ivermectine dans la peau.

Il s'avère que l'ivermectine est un composé présentant une instabilité chimique au contact de l'eau. Afin de la stabiliser, différentes solutions ont été apportées dans l'art antérieur : la demande de brevet EP 0 045 655 propose de former des micelles de tensio-actifs qui entourent l'ivermectine afin de la protéger de l'eau ; d'autres demandes, telles que WO 01/60380 ou WO 97/26895 proposent d'utiliser des solvants aqueux d'actifs tels que la N-méthyl-2-pyrrolidone. Enfin, les demandes WO2004/093886 et WO2005/089806 décrivent des émulsions comprenant une phase huileuse et une phase aqueuse, ladite phase aqueuse comprenant une phase active micellaire contenant l'ivermectine. Malheureusement ces concepts ne permettent pas une stabilité optimum de l'ivermectine.

Or la Demanderesse a mis au point une composition à base d'ivermectine, notamment sous forme d'émulsion huile-dans-eau, répondant parfaitement à ces attentes, comprenant une phase grasse dispersée dans une phase aqueuse, l'ivermectine étant solubilisée dans ladite phase grasse.

Cette formulation est une émulsion qui comprend de préférence un fort pourcentage d'eau.
Dans les compositions selon l'invention, l'ivermectine est entièrement solubilisée au sein de la phase huileuse interne de l'émulsion, et cette solubilisation dans les globules huileux de l'émulsion permet de limiter le contact de l'Ivermectine avec la phase aqueuse, que l'homme de l'art veillera à rendre exempte de solvants de l'Ivermectine. Une telle composition est de plus très bien tolérée. Cela permet avantageusement de garder un fort pourcentage d'eau, afin de conserver un produit aqueux adapté aux pathologies visées.
La présente invention a donc pour objet une composition pharmaceutique comprenant au moins une phase grasse, au moins une phase aqueuse et au moins un composé de la famille des avermectines, ledit composé de la famille des avermectines étant solubilisé dans ladite phase grasse. La phase grasse est donc la phase solvante de l'actif.

Par composition pharmaceutique, on entend une composition qui comprend des composés compatibles avec une application sur la peau, les muqueuses et/ou les phanères.

Les composés de la famille des avermectines utilisables selon l'invention sont choisis parmi l'ivermectine, l'invermectine, l'avermectine, l'abamectine, la doramectine, l'eprinomectine et la sélamectine. De préférence, le composé de la famille des avermectines est l'ivermectine.

De préférence, la composition selon l'invention comprend une seule phase aqueuse et une seule phase grasse et se présente sous forme d'émulsion huile-dans-eau, i.e. la phase grasse étant dispersée dans la phase aqueuse. Par émulsion huile-dans-eau, on entend selon l'invention un système constitué par un liquide (ici la phase grasse) se trouvant dispersé sous la forme de fines gouttelettes dans un autre liquide (ici la phase aqueuse), les deux liquides étant considérés comme insolubles ou très peu solubles l'un dans l'autre. La taille des particules est voisine de 1000 nm (1µm).

La composition selon l'invention est qualifiée d'émulsion stable en ce qu'elle présente une bonne stabilité physique et chimique dans le temps, même à une température supérieure à la température ambiante (par exemple 40°C), comme le montrent les exemples ci-après.

Dans les compositions selon l'invention, l'ivermectine est présente en quantité comprise entre 0.001 et 10%, de préférence entre 0.001 et 5% en poids par rapport au poids total de la composition, et de préférence entre 0.003 et 2%.

Dans les compositions selon l'invention, la phase aqueuse est présente en quantité comprise entre 30 et 95% en poids par rapport au poids total de la composition.

La phase grasse de la composition selon l'invention comprend au moins une phase grasse solvante de l'actif, ou phase huileuse solvante de l'actif, ou phase active. L'actif s'entend ici du composé de la famille des avermectines, de préférence l'ivermectine.

La phase huileuse solvante de l'actif comprend au moins un solvant huileux de l'actif distinct des huiles végétales et minérales. Ainsi, de préférence la phase huileuse solvante de l'actif comprend au moins un solvant huileux de l'actif choisi parmi les huiles synthétiques.

En effet, d'une part le principe actif, et notamment l'ivermectine, est mieux solubilisé dans les huiles synthétiques que dans les huiles minérales ou végétales. D'autre part, les compositions obtenues avec ce type de solvant présentent une stabilité améliorée.

La phase huileuse solvante de l'actif comprend ainsi au moins un solvant huileux de l'actif, choisi parmi le diisopropyl adipate vendu sous le nom de Crodamol DA

par la société Croda, le PPG 15 stearyl ether vendu sous le nom d'Arlamol E par la société Uniqema, l'octyl dodecanol vendu sous le nom d'Eutanol G par la société Cognis, le benzoate d'alkyle C12-C15 vendu sous le nom de Tegosoft TN par la société Degussa, et leurs mélanges.

Encore plus préférentiellement on utilisera le diisopropyl adipate ou le PPG-15 stearyl ether ou un mélange de ces deux composés.

De préférence, la phase active de la composition selon l'invention ne comprend pas de solvant distinct des solvants huileux décrits ci-dessus ni d'émulsionnant. En particulier, elle ne comprend pas de solvant de type alcool ou glycol. En effet, le(s) solvant(s) huileux décrit(s) ci-dessus suffi(sen)t à lui(eux) seul(s) à solubiliser le principe actif.

De préférence, la phase active selon l'invention contient uniquement au moins un solvant huileux décrit ci-dessus et au moins un composé de la famille des avermectines.

la phase grasse comprend au moins une phase huileuse solvante de l'actif (ou phase grasse solvante de l'actif). Elle peut également comprendre au moins une phase grasse non solvante de l'actif. De préférence, la phase grasse comprend une phase huileuse solvante de l'actif et une phase grasse non solvante de l'actif ; alternativement, de préférence, la phase grasse comprend uniquement une phase huileuse solvante de l'actif.
Par phase grasse non solvante de l'actif, on entend une phase lipophile qui comprend un ou des composés lipophiles non solvants de l'actif, ie dans laquelle les composés de la famille des avermectines ont une solubilité inférieure ou égale à 1% en poids par rapport au poids total de la phase grasse non solvante.

La phase grasse non solvante de l'actif comprend au moins un composé lipophile non solvant de l'actif, choisi notamment parmi des huiles non solvantes, des corps gras, des épaississants de la phase huileuse, et leurs mélanges.
Parmi les huiles non solvantes, on peut citer les huiles de silicone, dont la cyclométhicone, la diméthicone de viscosité entre 20 et 350 cst ; les huiles minérales, dont le Primol 352 et le Marcol 152 fabriqués par la société Esso, et leurs mélanges. Parmi les corps gras épaississants, on peut citer l'alcool stéarylique vendu sous le nom de Speziol C18 par Cognis ou l'alcool cétylique vendu sous le nom de Speziol C16 par la société Cognis, les cires, les beurres et leurs mélanges.

La composition selon l'invention comprend également au moins un émulsionnant, afin de stabiliser l'émulsion.
Ces émulsionnants sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides. Leur pouvoir émulsionnant est étroitement lié à la polarité de la molécule. Cette polarité est définie par le HLB (Balance Hydrophile/Lipophile).
Ces émulsionnants sont notamment choisis parmi le macrogol 21 stearylether vendu sous le nom de Brij 721 par Uniqema, le macrogol 2 stearylether vendu sous le nom de Brij 72P par Uniqema, le glyceryl/PEG 100 stearate vendu sous le nom d'Arlacel 165FL par Uniqema, le ceteareth 20 vendu sous le nom de Eumulgin B2 par la société Cognis, le PEG-6 and PEG 32 palmitostearate vendu sous le nom de TEFOSE 1500 par Gattefossé, le PEG 20 méthyl glucose sesquistearate vendu sous le nom de GLUCAMATE SSE 20 par Amerchol, des esters d'acides gras polyoxyéthylénés tels que l'Arlatone 983 non ionique de la société ICI ou le Methyl glucose sesquistearate vendu sous le nom de GLUCATE SS par Amerchol.

Ce type d'émulsionnant est utilisé à une concentration comprise entre 0.1 et 8% en poids, de préférence à une concentration comprise entre 1 et 8% en poids par rapport au poids total de la composition.

Les émulsions selon l'invention peuvent également comprendre des co-émulsionnants. Parmi ces composés, on trouve notamment les esters de sorbitan non ionique tels que l'oléate de sorbitan vendu sous la dénomination Arlacel 80 par la société ICI ou vendu sous le nom de Crill 4 par la société Croda, le sesquioléate de sorbitan vendu sous le nom de Arlacel 83 par la société ICI ou sous le nom de Montane 83 par la société SEPPIC, ou bien l'isostéarate de sorbitan ; les éthers d'alcools gras non ionique ayant un haut HLB, c'est-à-dire un HLB supérieur ou égal à 7 tels que le cétéareth-20 ou le cétéareth-12, ou les éthers d'alcools gras ayant un HLB bas, c'est-à-dire un HLB inférieur à 7, tel que le stéareth-2.

La composition selon l'invention peut également comprendre un gélifiant.
Comme gélifiants utilisables, on peut citer les carbomères vendus sous le nom de Carbopol 980 NF et Carbopol 981 NF par Noveon, le C10-30 alkyl acrylique cross polymère vendu sous le nom de Pemulen TR1 par la société Noveon, le gel d'acrylamide vendu sous le nom de Simulgel 600 par Seppic, les celluloses modifiées vendues sous le nom de Natrosol par la société Hercules-Aqualon ou Methocel par la société Dow chemical company, ou encore les biopolymères saccharidiques vendus sous le nom de Xantural par SPCI.

De préférence, la composition selon l'invention comprend :
0.01 à 25% de phase grasse solvante de l'actif
0 à 20% de phase grasse non solvante de l'actif
1 à 8% d'émulsionnant
0 à 5% d'un agent gélifiant
0.001 à 5% d'ivermectine
50 à 75% de phase aqueuse.

Encore plus préférentiellement, la composition selon l'invention comprend :
5 à 20% de phase grasse solvante de l'actif
0 à 10% de phase grasse non solvante de l'actif
2 à 5% d'émulsionnant
0 à 3% d'un agent gélifiant
0.001 à 2% d'ivermectine
55 à 70% de phase aqueuse.

La composition selon l'invention pourra en outre contenir des additifs usuellement utilisés dans le domaine cosmétique ou pharmaceutique, tels que
- des agents humectants tels que la glycérine, le sorbitol et le propylène glycol ;
- des agents conservateurs tels que le parahydroxybenzoate de méthyle, le parahydroxybenzoate de propyle, le parahydroxybenzoate de butyle, le phenoxyethanol, le chlorure de benzalkonium, l'alcool benzylique, le phenylethyl alcool, la chlorhexidine digluconate, la chlorephenesine ;
- des agents anti-irritants, tels que l'allantoïne, l'acide 18β glycyrrhétinique, le DL alpha tocophérol acétate ;
- des agents régulateurs d'humidité ;
- des agents régulateurs de pH, tels que l'acide citrique et l'hydroxyde de sodium ;
- des agents modificateurs de pression osmotique ;
- des filtres UV-A et UV-B ;
- et des antioxydants, tels que l'α-tocophérol, le butylhydroxyanisole (BHA) ou le butylhydroxytoluène (BHT), la vitamine E, le propyl gallate ou l'acide citrique.

Bien entendu, l'homme du métier saura adapter le choix des additifs ou les éventuels composés à ajouter à ces compositions et également le mode opératoire de telle manière que les propriétés avantageuses attachées intrinsèquement à la présente invention ne soient pas ou substantiellement pas altérées par l'addition envisagée.
Ces additifs peuvent être présents dans la composition de 0.001 à 20% en poids par rapport au poids total de la composition.

L'invention a également pour objet un procédé de préparation de la composition, qui comprend les étapes suivantes :
a) mélange de l'ivermectine avec au moins un solvant huileux, jusqu'à ce que l'ivermectine soit solubilisée, afin de former la phase grasse ;
b) mélange des constituants de la phase aqueuse, jusqu'à homogénéité ;
c) incorporation de la phase grasse dans la phase aqueuse pour former une émulsion.

La phase grasse peut comprendre une phase huileuse solvante de l'actif et une phase grasse non solvante de l'actif. Dans ce cas, le procédé comprend entre les étapes a) et b) ci-dessus une étape de mélange de la phase huileuse obtenue en a) avec au moins un composé lipophile (complémentaire) non solvant de l'actif, ledit composé étant notamment tel que décrit ci-dessus.

L'invention a également pour objet l'utilisation de la composition selon l'invention pour la fabrication d'une préparation pharmaceutique destinée à traiter les affections dermatologiques.

Par affections dermatologiques, on entend plus particulièrement la rosacée, l'acné vulgaire, la dermite séborrhéique, la dermatite périorale, les éruptions acnéiformes, la dermatite acantholytique transitoire, et l'acné *miliaris necrotica*.

La composition selon l'invention est particulièrement adaptée au traitement de la rosacée.

Il va être maintenant donné, à titre d'illustration et sans aucun caractère limitatif, diverses formulations de type émulsion comprenant de l'ivermectine selon l'invention. Les quantités y sont données en % en poids, sauf mention contraire.

### EXEMPLES

### Exemple 1 : ETUDE DE SOLUBILITE 1 STABILITE DE L'ACTIF

Solubilité maximale de l'Ivermectine à T1 heure à Température Ambiante (TA) dans différents excipients de phase huileuse et stabilité 1 mois à TA et T40°C

| | % (W/W) | Stabilité 1 mois à TA | Stabilité 1 mois à 40°C |
|---|---|---|---|
| Diisopropyl adipate | 10.4 | Stable | stable |
| PPG 15 stearyl ether | 3.3 | Stable | Stable |
| Alcool de guerbet | 2.5 | Non testé | Non testé |
| benzoate d'alkyle C12-15 | 1.6 | Non testé | Non testé |

### Exemple 2:

### Composition 1

| Phases | Nom INCI | % formulaire |
|---|---|---|
| A | Eau purifiée | Qsp 100% |
| A | Glycerol | 7.00 |
| A | Parahydroxybenzoate de méthyle | 0.20 |
| A | Edetate de disodium | 0.10 |
| A | Allantoine | 0.20 |
| A | Carbomère | 0.15 |
| B | Macrogol 21 stearyl ether | 3.00 |
| B | Glyceryl/PEG 100 stearate | 3.00 |
| B | Propyl parahydroxybenzoate | 0.10 |
| B | Stearyl alcool | 2.00 |
| B | Butylhydroxytoluene | 0.10 |
| C | Diisopropyl adipate | 15.00 |
| C | Ivermectine | 1.00 |
| D | Cyclopentasiloxane | 6.00 |
| E | Acrylamide/Sodium Acryloyldimethyl Taurate Copolymère & Isohexadecane & Polysorbate 80 | 1.00 |
| F | Hydroxyde de sodium (sol à 10%) | Qsp ph 6.3 |

### Mode opératoire de la composition 1

Fabrication sous lumière inactinique

### Préparation des phases :

### Phase A

Incorporer les matières premières de la phase A dans un bécher et chauffer à 75°C.

### Phase B

Incorporer les matières premières de la phase B puis chauffer à 75°C.

### Phase active C

Solubiliser l'actif dans l'huile solvante et ajouter la phase active C à la phase B.

### Emulsification et neutralisation :

Emulsionner en introduisant la phase B dans la phase A sous agitation Rayneri.
A 50°C introduire les phases D puis E.
A température ambiante, neutraliser avec la solution de soude afin d'obtenir un pH de 6.3 puis homogénéiser.

### Exemple 3:

### Composition 2

| Phases | Nom INCI | % formulaire |
|---|---|---|
| A | Eau purifiée | Qsp 100% |
| A | Methyl parahydroxybenzoate | 0.15 |
| A | Glycerol | 7.00 |
| A | Edetate de disodium | 0.10 |
| A | Allantoine | 0.20 |
| A | Carbomère | 0.30 |
| B | Macrogol 2 stearyl ether | 2.50 |
| B | Macrogol 21 stearyl ether | 2.50 |
| B | PPG 15 stearyl ether | 4.00 |
| B | Propyl parahydroxybenzoate | 0.05 |
| B | Butylhydroxytoluene | 0.10 |
| C | Diisopropyl adipate | 16.00 |
| C | Ivermectine | 1.00 |
| D | Dimethicone | 1.00 |
| E | Hydroxyde de sodium (sol à 10%) | Qsp ph 6.3 |

Le mode opératoire de la composition 2 est identique à celui de la composition 1 (exemple 3).

### Exemple 4:

### Composition 3

| Phases | Nom INCI | % formulaire |
|---|---|---|
| A | Eau purifiée | Qsp 100% |
| A | Methyl parabène | 0.20 |
| A | Glycerol | 7.00 |
| A | Edetate de disodium | 0.10 |
| A | Allantoine | 0.20 |
| A | Carbomère | 0.30 |
| B | Macrogol 2 stearylether | 2.50 |
| B | Macrogol 21 stearyl ether | 2.50 |
| B | PPG 15 stearyl ether | 4.00 |
| B | Propyl parabène | 0.10 |
| B | Butyl hydroxytoluene | 0.10 |
| C | Diisopropyl adipate | 16.00 |
| C | Ivermectine | 1.00 |
| D | Dimethicone 200 | 1.00 |
| E | Chlorure de benzalkonium | 0.05 |
| E | Eau purifiée | 5.00 |
| F | Hydroxyde de sodium (sol à 10%) | Qsp ph 6.3 |

Le mode opératoire de la composition 3 est identique à celui de la composition 2 (exemple 3).

### Exemple 5:

### Composition 4

| Phases | Nom INCI | % formulaire |
|---|---|---|
| A | Eau purifiée | Qsp 100% |
| A | Methyl paraben | 0.20 |
| A | Glycerol | 7.00 |
| A | Disodium edetate | 0.10 |
| A | Allantoine | 0.20 |
| A | Carbomer | 0.30 |
| B | Macrogol 2 stearylether | 2.50 |
| B | Ceteareth 20 | 2.50 |
| B | Propyl paraben | 0.10 |
| C | Diisopropyl adipate | 16.00 |
| C | Ivermectine | 1.00 |
| D | Dimethicone 200 | 1.00 |
| E | Benzalkonium chloride | 0.020 |
| F | Sodium hydroxyde (sol à 10%) | Qsp ph 6.3 |

Le mode opératoire de la composition 4 est identique à celui de la composition 3 (exemple 4).

### Exemple 6:

### Composition 5

| Phases | Nom INCI | % formulaire |
|---|---|---|
| A | Eau purifiée | Qsp 100% |
| A | Glycerol | 7.00 |
| A | Edetate de disodium | 0.10 |
| A | Phenoxyethanol | 1.00 |
| A | Natrosol | 0.50 |
| B | Glyceryl/PEG 100 stearate | 5.00 |
| C | Octyldodecanol | 20.00 |
| C | Benzoate d'alkyle C12-15 | 5.00 |
| C | Ivermectine | 0.50 |
| F | Hydroxyde de sodium (sol à 10%) | Qsp ph 6.3 |

Le mode opératoire de la composition 5 est identique à celui de la composition 4 (exemple 5).

### Exemple 7 :

### Composition 6

| Phases | Nom INCI | % formulaire |
|---|---|---|
| A | Eau purifiée | Qsp 100% |
| A | Methyl paraben | 0.20 |
| A | Glycerol | 7.00 |
| A | Disodium edetate | 0.10 |
| A | Allantoine | 0.20 |
| A | Carbomer | 0.30 |
| A | Phenoxyethanol | 1.00 |
| B | Macrogol 2 stearylether | 2.50 |
| B | Macrogol 21 stearylether | 2.50 |
| B | Propyl paraben | 0.10 |
| B | Butyl hydroxytoluene | 0.10 |
| B | PPG-15 stearyl ether | 4.00 |
| C | Diisopropyl adipate | 16.00 |
| C | Ivermectine | 1.00 |
| D | Dimethicone 200 | 1.00 |
| F | Sodium hydroxyde (sol à 10%) | Qsp ph 6.3 |

Le mode opératoire de la composition 6 est identique à celui de la composition 5 (exemple 6).

### Exemple 8 : Stabilités physique et chimique

La stabilité physique des formulations selon l'invention est mesurée par une observation macroscopique et microscopique de la formulation à température ambiante (TA) (20-30°C), 40°C et 4°C à T1 mois, T2 mois, T3 mois et T6 mois.
A TA, l'observation macroscopique permet de garantir l'intégrité physique des produits.

On complète la caractérisation du produit fini par une mesure du seuil d'écoulement. On utilise un rhéomètre HAAKE de type VT550 avec un mobile de mesure SVDIN.
Les rhéogrammes sont réalisés à 25°C et à la vitesse de cisaillement de 4 s-1 (γ), et en mesurant la contrainte de cisaillement. Par seuil d'écoulement (τ0 exprimé en Pascal) on entend la force nécessaire (contrainte de cisaillement minimum) pour vaincre les forces de cohésion de type Van der Waals et provoquer l'écoulement. Le seuil d'écoulement est assimilé à la valeur trouvée à la vitesse de cisaillement de 4s-1.
Ces mesures sont réalisées à T24h, à T 1, T2, T3 et T6 mois.

La stabilité chimique des compositions est également mesurée par dosage de l'actif Ivermectine en HPLC à TA et 40°C à T0, T1, T2, T3 et T6 mois. Elle est comparée à celle obtenue avec les formules crème issue de la demande WO2004/093886 et gel-crème issue de la demande WO2005/089806 suivantes :
Résultat obtenu : R en %

### Composition 1

| | | |
|---|---|---|
| Dosage T0: analytique 101.7% | Aspect macroscopique pH 24h :6.3 | Lait |
| | Viscosité : τ₍₄ₛ₋₁₎ en Pa.s-1 | 28 |

| | | **T1 mois** | **T2 mois** | **T3 mois** | **T6 mois** |
|---|---|---|---|---|---|
| **TA** | **Viscosité :** τ ₍₄ₛ₋₁₎ en Pa.s-1 | 35 | 39 | 42 | 51 |
| | **Aspect macroscopique** | Conforme | Conforme | Conforme | Conforme |
| | **pH** | 6.1 | 6.1 | 6.1 | 6.0 |
| | **Aspect microscopique** | Conforme | Conforme | Conforme | Conforme |
| | **Dosage analytique** | 99.8% | 100.1% | 100.8% | 102.3% |
| **4°C** | **Aspect macroscopique** | Conforme | Conforme | Conforme | Conforme |
| | **Aspect microscopique** | Conforme | Conforme | Conforme | Conforme |
| **40°C** | **Aspect macroscopique** | Conforme | Conforme | Conforme | Conforme |
| | **Aspect microscopique** | Conforme | Conforme | Conforme | Conforme |
| | **Dosage analytique** | 101% | 99.7% | 100.8% | 104.7% |

### Composition 2

| | | |
|---|---|---|
| Dosage T0: analytique 93.6% | Aspect macroscopique pH 24h: 6.1 | Crème souple |
| | Viscosité : τ₍₄ₛ₋₁₎ en Pa.s-1 | 56 |

| | | **T1 mois** | **T2 mois** | **T3mois** | **T6 mois** |
|---|---|---|---|---|---|
| **TA** | **Viscosité** : τ₍₄₁₋₁₎ en Pa.s-1 | 65 | 70 | 64 | 63 |
| | **Aspect macroscopique** | Conforme | Conforme | Conforme | Conforme |
| | **pH** | 6.1 | 6.1 | 6.1 | 6.0 |
| | **Aspect microscopique** | Conforme | Conforme | Conforme | Conforme |
| | **Dosage analytique** | 93.6% | 93.9% | 93.9% | 94.4% |
| **4°C** | **Aspect macroscopique** | Conforme | Conforme | Conforme | Conforme |
| | **Aspect microscopique** | Conforme | Conforme | Conforme | Conforme |
| | **Aspect macroscopique** | Conforme | Conforme | Conforme | Conforme |
| **40°C** | **Aspect microscopique** | Conforme | Conforme | Conforme | Conforme |
| | **Dosage analytique** | 92.7% | 94.8% | 91.9% | 94.5% |

### Composition 3

| | | |
|---|---|---|
| Dosage T0: analytique 99.8% | Aspect macroscopique pH 24h: 6.2 | Lait épais |
| | Viscosité : τ ₍₄ₛ₋₁₎ en Pa.s-1 | 66 |

| | | **T1 mois** | **T2 mois** | **T3 mois** | **T6 mois** |
|---|---|---|---|---|---|
| | **Viscosité** : τ₍₄ₛ₋₁₎ en Pa.s-1 | 58 | 69 | 72 | 61 |
| **TA** | **Aspect macroscopique** | Conforme | Conforme | Conforme | Conforme |
| | **pH** | 6.1 | 6.0 | 6.0 | 6.0 |
| | **Aspect microscopique** | Conforme | Conforme | Conforme | Conforme |
| | **Dosage analytique** | 102.0% | 102.4% | 102.8% | 102.7% |
| **4°C** | **Aspect macroscopique** | Conforme | Conforme | Conforme | Conforme |
| | **Aspect microscopique** | Conforme | Conforme | Conforme | Conforme |
| | **Aspect macroscopique** | Conforme | Conforme | Conforme | Conforme |
| **40°C** | **Aspect microscopique** | Conforme | Conforme | Conforme | Conforme |
| | **Dosage analytique** | 102.4% | 101.7% | 104.2% | 100.5% |

### Composition 4

| | | |
|---|---|---|
| Dosage T0:95.2 analytique | Aspect macroscopique pH 24h: 6.0 | Lait brillant blanc |
| | Viscosité : τ₍₄ₛ₋₁₎ en Pa.s-1 | 38 |

| | | **T1 mois** | **T2 mois** | **T3 mois** | **T6 mois** |
|---|---|---|---|---|---|
| | **Viscosité** : τ₍₄₁₋₁₎ en Pa.s-1 | 35 | 34 | 41 | 34 |
| | **Aspect macroscopique** | 6.0 | 6.0 | 6.0 | 5.9 |
| | **pH** | | | | |
| **TA** | **Aspect microscopique** | Conforme | Conforme | Conforme | Conforme |
| | **Dosage analytique** | 95.2% | NR | 100.4% | 94.4% |
| **4°C** | **Aspect macroscopique** | Conforme | Conforme | Conforme | Conforme |
| | **Aspect microscopique** | Conforme | Conforme | Conforme | Conforme |
| | **Aspect macroscopique** | Conforme | Conforme | Conforme | Conforme |
| **40°C** | **Aspect microscopique** | Conforme | Conforme | Conforme | Conforme |
| | **Dosage analytique** | 104.2% | NR | 101.0% | 98.3% |

### Composition 7 (composition 1 avec actif à 0.03%)

| | | |
|---|---|---|
| Dosage T0: analytique 93.5% | Aspect macroscopique pH 24h : 6.7 | Lait |
| | Viscosité : τ ₍₄ₛ₋₁₎ en Pa.s-1 | 26 |

| | | **T1 mois** | **T2 mois** | **T3 mois** | **T6 mois** |
|---|---|---|---|---|---|
| **TA** | **Viscosité** : τ₍₄ₛ₋₁₎ en Pa.s-1 | 27 | 31 | 20 | NR |
| | **Aspect macroscopique** | Conforme | Conforme | Conforme | Conforme |
| | **pH** | 6.5 | 6.5 | 6.5 | 6.4 |
| | **Aspect microscopique** | Conforme | Conforme | Conforme | Conforme |
| | **Dosage analytique** | 98.4% | 98.3% | 97.4% | 100.6% |
| **4°C** | **Aspect macroscopique** | Conforme | Conforme | Conforme | Conforme |
| | **Aspect microscopique** | Conforme | Conforme | Conforme | Conforme |
| | **Aspect macroscopique** | Conforme | Conforme | Conforme | Conforme |
| **40°C** | **Aspect microscopique** | Conforme | Conforme | Conforme | Conforme |
| | **Dosage analytique** | 98.6% | 99.5% | 101.0% | 97.8% |

### Composition 8 (composition 2 avec actif à 0.03%)

| | | |
|---|---|---|
| Dosage T0: analytique 97.6% | Aspect macroscopique pH 24h: 6.2 | Crème souple |
| | Viscosité : τ ₍₄ₛ₋₁₎ en Pa.s-1 | 54 |

| | | **T1 mois** | **T2 mois** | **T3 mois** | **T6 mois** |
|---|---|---|---|---|---|
| | **Viscosité** : τ₍₄ₛ₋₁₎ en Pa.s-1 | NA | 77 | 80 | 83 |
| **TA** | **Aspect macroscopique** | Conforme | Conforme | Conforme | Conforme |
| | **pH** | NA | 6.3 | 6.3 | 6.2 |
| | **Aspect microscopique** | Conforme | Conforme | Conforme | Conforme |
| | **Dosage analytique** | 95.9% | 96.0% | 95.5% | 97.3% |
| **4°C** | **Aspect macroscopique** | Conforme | Conforme | Conforme | Conforme |
| | **Aspect microscopique** | Conforme | Conforme | Conforme | Conforme |
| | **Aspect macroscopique** | Conforme | Conforme | Conforme | Conforme |
| **40°C** | **Aspect microscopique** | Conforme | Conforme | Conforme | Conforme |
| | **Dosage analytique** | 95.9% | 106.0% (Récipient non étanche) | 95.7% | 96.3% |

### Exemple 9 : Composition

| Phases | Nom INCI | % formulaire |
|---|---|---|
| A | Eau purifiée | Qsp 100% |
| A | Methyl paraben | 0.20 |
| A | Glycerol | 7.00 |
| A | Edetate de disodium | 0.10 |
| A | Allantoine | 0.20 |
| A | Carbomer | 0.30 |
| B | Macrogol 2 stearylether | 2.50 |
| B | Macrogol 21 stearyl ether | 2.50 |
| B | Propyl paraben | 0.10 |
| B | Butyl hydroxytoluene | 0.10 |
| C | Diisopropyl adipate | 16.00 |
| C | Ivermectine | 1.00 |
| D | Dimethicone 200 | 1.00 |
| E | Chlorure de benzalkonium | 0.02 |
| E | Eau purifiée | 5.00 |
| F | Hydroxyde de sodium (sol à 10%) | Qsp ph 6.3 |

### Stabilités physique et chimique

| | | |
|---|---|---|
| Dosage T0: analytique 96.8% | Aspect macroscopique pH 24h: 6.2 | Lait épais |
| | Viscosité : τ₍₄ₛ₋₁₎ en Pa.s-1 | 66 |

| | | **T1mois** | **T3mois** | **T6mois** |
|---|---|---|---|---|
| **TA** | **Viscosité** : τ₍₄ₛ₋₁₎ | 84 | 79 | NA |
| | en Pa.s-1 | | | |
| | **Aspect macroscopique** | Conforme | Conforme | Conforme |
| | **pH** | 6.2 | 6.1 | 6.1 |
| | **Aspect microscopique** | Conforme | Conforme | Conforme |
| | **Dosage analytique** | 99.2% | 99.1% | 100% T7mois |
| **4°C** | **Aspect macroscopique** | Conforme | Conforme | Conforme |
| | **Aspect microscopique** | Conforme | Conforme | Conforme |
| | **Aspect macroscopique** | Conforme | Conforme | Conforme |
| **40°C** | **Aspect microscopique** | Conforme | Conforme | Conforme |
| | **Dosage analytique** | 99.1% | 98.9% | 98.9% T7mois |

### Exemple 10 : Composition

| Phases | Nom INCI | % formulaire |
|---|---|---|
| A | Eau purifiée | Qsp 100% |
| A | Methyl paraben | 0.20 |
| A | Glycerol | 7.00 |
| A | Edetate de disodium | 0.10 |
| A | Allantoine | 0.20 |
| A | Carbomer | 0.30 |
| B | Macrogol 2 stearylether | 2.50 |
| B | Macrogol 21 stearyl ether | 2.50 |
| B | PPG 15 stearyl ether | 4.00 |
| B | Propyl paraben | 0.10 |
| B | Butyl hydroxytoluene | 0.10 |
| C | Diisopropyl adipate | 16.00 |
| C | Ivermectine | 1.00 |
| D | Dimethicone 200 | 1.00 |
| E | Chlorure de benzalkonium | 0.02 |
| E | Eau purifiée | 5.00 |
| F | Hydroxyde de sodium (sol à 10%) | Qsp ph 6.3 |

### Stabilités physique et chimique

| | | |
|---|---|---|
| Dosage T0: analytique 98.9% | Aspect macroscopique pH 24h: 6.1 | Lait épais |
| | Viscosité : τ ₍₄ₛ₋₁₎ en Pa.s-1 | 74 |

| | | **T1mois** | **T3mois** | **T6mois** |
|---|---|---|---|---|
| **TA** | **Viscosité:** τ_{(4s-1) en Pa.s-1} | 90 | 80 | NA |
| | **Aspect macroscopique** | Conforme | Conforme | Conforme |
| | **pH** | 6.1 | 6.1 | 6.1 |
| | **Aspect microscopique** | Conforme | Conforme | Conforme |
| | **Dosage analytique** | 100.6% | 102.5% | 103.6% T7mois |
| **4°C** | **Aspect macroscopique** | Conforme | Conforme | Conforme |
| | **Aspect microscopique** | Conforme | Conforme | Conforme |
| | **Aspect macroscopique** | Conforme | Conforme | Conforme |
| **40°C** | **Aspect microscopique** | Conforme | Conforme | Conforme |
| | **Dosage analytique** | 100.7% | 99.5% | 102.3% T7mois |

### Exemple 11 : Composition

| Phases | Nom INCI | % formulaire |
|---|---|---|
| A | Eau purifiée | Qsp 100% |
| A | Methyl paraben | 0.20 |
| A | Glycerol | 7.00 |
| A | Edetate de disodium | 0.10 |
| A | Allantoine | 0.20 |
| A | Carbomer | 0.30 |
| B | Macrogol 2 stearylether | 2.50 |
| B | Steareth 20 | 2.50 |
| B | Propyl paraben | 0.10 |
| B | Butyl hydroxytoluene | 0.10 |
| C | Diisopropyl adipate | 16.00 |
| C | Ivermectine | 1.00 |
| D | Dimethicone 200 | 1.00 |
| E | Chlorure de benzalkonium | 0.02 |
| E | Eau purifiée | 5.00 |
| F | Hydroxyde de sodium (sol à 10%) | Qsp ph 6.3 |

### Stabilités physique et chimique

| | | |
|---|---|---|
| Dosage T0: analytique 100.1% | Aspect macroscopique pH 24h: 6.0 | Lait épais |
| | Viscosité : τ ₍₄ₛ₋₁₎ en Pa.s-1 | 56 |

| | | T1mois | **T3mois** | **T6mois** |
|---|---|---|---|---|
| **TA** | **Viscosité :** τ₍₄₁₋₁₎ en Pa.s-1 | 50 | 48 | NA |
| | **Aspect macroscopique** | Conforme | Conforme | Conforme |
| | **pH** | 6.0 | 6.0 | 6.0 |
| | **Aspect microscopique** | Conforme | Conforme | Conforme |
| | **Dosage analytique** | 100.1% | 99.9% | 99.6% |
| **4°C** | **Aspect macroscopique** | Conforme | Conforme | Conforme |
| | **Aspect microscopique** | Conforme | Conforme | Conforme |
| | **Aspect macroscopique** | Conforme | Conforme | Conforme |
| **40°C** | **Aspect microscopique** | Conforme | Conforme | Conforme |
| | **Dosage analytique** | 99.6% | 99.9% | 101.8% |

**Exemple 12: Comparaison des stabilités chimiques des émulsions selon l'invention versus les stabilités chimiques des crèmes décrites dans la demande de brevet** WO2004/093886

Les compositions A et B sont des compositions telles que décrites dans la demande de brevet WO2004/093886.

**Composition A**

| Phases | Fonction | % Formulaire |
|---|---|---|
| Phase grasse | Huile émolliente | 4.00 |
| | Emulsionnants | 5.00 |
| | Alcool gras | 6.00 |
| | silicone | 0.50 |
| | Conservateur | 0.10 |
| Phase aqueuse | Eau purifiée | Qsp 100% |
| | Gélifiant | 0.20 |
| | Humectant | 4.00 |
| | Antioxydant | 0.05 |
| | Chélatant | 0.05 |
| | Conservateur | 0.20 |
| Neutralisant | Base | Qsp pH 6.30 |
| Phase active | Oleyl alcohol | 2.00 |
| | Propylene Glycol | 2.00 |
| | Phenoxyethanol | 1.00 |
| | **Ivermectine** | 1.00 |

**Composition B**

| Phases | Fonction | % Formulaire |
|---|---|---|
| Phase grasse | Huile émolliente | 4.00 |
| | Emulsionnants | 5.00 |
| | Acide gras | 2.00 |
| | Cire autoémulsionnable | 1.00 |
| | Silicone | 0.50 |
| | Conservateur | 0.10 |
| Phase aqueuse | Eau purifiée | Qsp 100% |
| | Gélifiant | 1.00 |
| | Humectant | 4.00 |
| | Antioxydant | 0.05 |
| | Chélatant | 0.05 |
| | Conservateur | 0.70 |
| Neutralisant | Base | Qsp pH 6.30 |
| Phase active | Glycérol triacetate | 1.00 |
| | Propylene Glycol | 4.00 |
| | Phenoxyethanol | 0.50 |
| | **Ivermectine** | 1.00 |

| ***Dosage analytique*** | | **T0** | **T1 mois** | **T2 mois** | **T3 mois** | **T6 mois** | **T9 mois** | **Perte en %** |
|---|---|---|---|---|---|---|---|---|
| ***Composition A*** | TA | 101.0% | 100.1% | 99.3% | 98.6% | 95.5% | 91.3% | +/-10% |
| ***Composition B*** | | 99.8% | 88.60% | 83.5% | 77.0% | 79.9% | 69.9% | +/-30% |
| ***Composition 3 selon l'invention*** | TA | 99.8% | 102.0% | 102.4% | 102.8% | 102.7% | Non réalisé | 0% |
| ***Composition 5 selon l'invention*** | | 93.5% | 98.4% | 98.3% | 97.4% | 100.6% | 100.9% | 0% |

Les résultats obtenus avec les formulations selon l'invention montrent bien que l'ivermectine solubilisée dans une huile solvante de la phase grasse, est beaucoup plus stable à température ambiante (TA) (25°C) que dans les formulations crèmes où l'ivermectine est solubilisée dans une phase micellaire.

**Exemple 13: Comparaison des stabilités chimiques des émulsions selon l'invention versus les stabilités chimiques des gels crèmes décrits dans la demande de brevet** WO2005/089806

Les compositions C et D sont des gels crème telles que décrites dans la demande de brevet WO2005/089806.

### Composition C

| Phases | Fonction | % Formulaire |
|---|---|---|
| Phase grasse | Huile émolliente | 10.00 |
| | Co-émulsionnants | 1.00 |
| | Antioxydant | 0.20 |
| | Conservateur | 0.10 |
| Phase aqueuse | Eau purifiée | Qsp 100% |
| | Gélifiant | 0.45 |
| | Humectant | 7.00 |
| | Chélatant | 0.10 |
| | Hydratant | 0.20 |
| Neutralisant | Base | Qsp pH 6.30 |
| Phase active | Polysorbate 80 | 4.00 |
| | Propylene Glycol | 4.00 |
| | Alcool benzylique | 1.00 |
| | **Ivermectine** | **0.10** |

### Composition D

| Phases | Fonction | % Formulaire |
|---|---|---|
| Phase grasse | Huile émolliente | 10.00 |
| | Co-émulsionnants | 1.00 |
| | Antioxydant | 0.20 |
| | Conservateur | 0.10 |
| Phase aqueuse | Eau purifiée | Qsp 100% |
| | Gélifiant | 0.45 |
| | Humectant | 5.00 |
| | Chélatant | 0.10 |
| | Hydratant | 0.20 |
| Neutralisant | Base | Qsp pH 6.30 |
| Phase active | Polysorbate 80 | 4.00 |
| | Propylene Glycol | 4.00 |
| | Alcool benzylique | 3.00 |
| | **Ivermectine** | **0.03** |

| ***Dosage analytique*** | | **T0** | **T1 mois** | **T2 mois** | **T3 mois** | **T6 mois** | **Perte en %** |
|---|---|---|---|---|---|---|---|
| ***Composition C*** | 40°C | 99.1% | 97.2% | 98.0% | 94.5% | 95.8% | +/-3% |
| ***Composition D*** | | 98.0% | 93.1% | 90.6% | 106.9* | 91.1% | +/-7% |
| ***Composition 3 selon l'invention*** | 40°C | 99.8% | 102.4 % | 101.7% | 104.2*% | 100.5% | 0% |
| ***Composition 5 selon l'invention*** | | 93.5% | 98.6% | 99.5% | 101.0% | 97.8% | 0% |

### *Problème d'étanchéité du contenant

Les résultats obtenus avec les formulations selon l'invention montrent bien que l'ivermectine solubilisée dans une huile solvante de la phase grasse est beaucoup plus stable à 40°C que dans les gels-crèmes où l'ivermectine est solubilisée dans une phase micellaire. L'ivermectine reste cependant stable dans les deux types de formulation aux autres températures (4°C et T ambiante).

### Exemple 14 : Etude de libération-pénétration :

L'étude de libération-pénétration est réalisée avec les 3 formules suivantes :
- crème de référence : composition A de l'exemple 9,
- gel crème : composition C de l'exemple 10 à 1% d'ivermectine.
- émulsion selon l'invention : composition 4 selon l'invention.

But : comparer l'absorption percutanée in vitro de l'ivermectine radiomarquée à travers la peau humaine à 0, 1 % (m/m) dans les 3 formulations.

La quantité d'ivermectine dans l'épiderme et le stratum corneum est respectivement de 0.56% pour la formule crème de référence (composition A), de 0.65% pour la formule gel-crème (composition C à 1% d'ivermectine) et de 0.97% pour l'émulsion selon l'invention (composition 4).
A partir de ces résultats, on peut déduire qu'il existe un effet de formulation sur la libération/pénétration de l'ivermectine, même si cette dernière a un poids moléculaire élevé.

L'émulsion selon l'invention permet d'avoir une meilleure libération-pénétration de l'actif ivermectine que celle obtenue avec la crème de référence ou le gel crème

### Exemple 15: Etude de tolérance locale:

Une étude de tolérance a été menée sur des placebos des formulations crème de référence (composition A de l'exemple 9), gel-crème (composition C de l'exemple 11 avec 1% de phénoxyéthanol à la place de l'alcool benzylique) et émulsion selon l'invention (composition 6 de l'exemple 7).
Traitement : application quotidienne du jour 1 au jour 6 de 20µl de la formulation sur l'oreille droite chez la souris Balb/c.

Méthode d'évaluation : Observation clinique et mesure de l'épaisseur de l'oreille de souris du jour 2 au jour 12.
Pesée des animaux le jour 1 et le jour 12.

Les 3 placebos testés crème de référence, gel-crème et émulsion selon l'invention augmentent faiblement l'épaisseur de l'oreille, respectivement de 14%, 8% et 9% entre la période jour 2-jour 12. Les formulations crème de référence, gel-crème et émulsion selon l'invention sont très peu irritantes chez la souris et ne devraient pas être irritantes chez l'homme.

## Revendications

1. Composition pharmaceutique comprenant au moins une phase grasse, au moins une phase aqueuse et au moins un composé de la famille des avermectines, **caractérisée en ce que** la phase grasse comprend au moins un solvant huileux choisi parmi le diisopropyl adipate, le PPG 15 stearyl ether, l'octyl dodecanol, le benzoate d'alkyle C12-C15 et leurs mélanges, dans lequel le composé de la famille des avermectines est solubilisé.

2. Composition pharmaceutique selon la revendication 1, **caractérisée en ce que** le composé de la famille des avermectines est choisi parmi l'ivermectine, l'invermectine, l'avermectine, l'abamectine, la doramectine, l'eprinomectine et la sélamectine.

3. Composition pharmaceutique selon l'une des revendications 1 ou 2, **caractérisée en ce que** le composé de la famille des avermectines est l'ivermectine.

4. Composition selon l'une des revendications 1 à 3, **caractérisée en ce qu'**elle se présente sous forme d'émulsion huile-dans-eau.

5. Composition selon l'une des revendications précédentes, **caractérisée en ce que** la quantité d'ivermectine représente de 0.001 à 10% et de préférence de 0.001 à 5% en poids par rapport au poids total de la composition.

6. Composition selon l'une des revendications 1 à 5, **caractérisée en ce que** la phase grasse comprend en outre une phase grasse non solvante de l'actif dans laquelle le composé de la famille des avermectines a une solubilité inférieure ou égale à 1% en poids par rapport au poids total de la phase grasse non solvante.

7. Composition selon la revendication 6, **caractérisée en ce que** la phase grasse non solvante de l'actif est choisie parmi les huiles de silicone, les huiles minérales, l'alcool stéarylique, l'alcool cétylique, les cires, les beurres et leurs mélanges.

8. Composition selon l'une des revendications 1 à 7, **caractérisée en ce que** la phase aqueuse est présente en quantité comprise entre 30 et 95% en poids par rapport au poids total de la composition.

9. Composition selon l'une des revendications 1 à 8, **caractérisée en ce qu'**elle comprend :
0.01 à 25% de phase grasse solvante de l'actif
0 à 20% de phase grasse non solvante de l'actif
1 à 8% d'émulsionnant
0 à 5% d'un agent gélifiant
0.001 à 5% d'ivermectine
50 à 75% de phase aqueuse.

10. Composition selon l'une des revendications 1 à 9, **caractérisée en ce qu'**elle comprend :
5 à 20% de phase grasse solvante de l'actif
0 à 10% de phase grasse non solvante de l'actif
2 à 5% d'émulsionnant
0 à 3% d'un agent gélifiant
0.001 à 2% d'ivermectine
55 à 70% de phase aqueuse.

11. Procédé de préparation d'une composition selon l'une des revendications 1 à 10, comprenant les étapes suivantes :
a) mélange de l'ivermectine avec au moins un solvant huileux, jusqu'à ce que l'ivermectine soit solubilisée, afin de former la phase grasse ;
b) mélange des constituants de la phase aqueuse, jusqu'à homogénéité ;
c) incorporation de la phase grasse dans la phase aqueuse pour former une émulsion.

12. Procédé selon la revendication 11, **caractérisé en ce qu'**il comprend entre les étapes a) et b) une étape de mélange de la phase grasse avec au moins un composé lipophile non solvant de l'actif.

13. Utilisation d'une composition selon l'une des revendications 1 à 10 pour la fabrication d'une préparation pharmaceutique destinée à traiter la rosacée, l'acné vulgaire, la dermite séborrhéique, la dermatite périorale, les éruptions acnéiformes, la dermatite acantholytique transitoire, et l'acné miliaris necrotica.

14. Utilisation selon la revendication 13, **caractérisée en ce que** la préparation pharmaceutique est destinée à traiter la rosacée.

## Patentansprüche

1. Pharmazeutische Zusammensetzung, die mindestens eine Fettphase, mindestens eine wässrige Phase und mindestens eine Verbindung der Familie der Avermectine umfasst, **dadurch gekennzeichnet, dass** die Fettphase mindestens ein öliges Lösungsmittel, ausgewählt aus Diisopropyladipat, PPG-15-Stearylether, Octyldodecanol, C12-C15-Alkylbenzoat und ihren Mischungen, in dem die Verbindung der Familie der Avermectine gelöst ist, umfasst.

2. Pharmazeutische Zusammensetzung nach Anspruch 1, **dadurch gekennzeichnet, dass** die Verbindung der Familie der Avermectine aus Ivermectin, Invermectin, Avermectin, Abamectin, Doramectin, Eprinomectin und Selamectin ausgewählt ist.

3. Pharmazeutische Zusammensetzung nach einem der Ansprüche 1 oder 2, **dadurch gekennzeichnet, dass** es sich bei der Verbindung der Familie der Avermectine um Ivermectin handelt.

4. Zusammensetzung nach einem der Ansprüche 1 bis 3, **dadurch gekennzeichnet, dass** sie in Form einer Öl-in-Wasser-Emulsion vorliegt.

5. Zusammensetzung nach einem der vorhergehenden Ansprüche, **dadurch gekennzeichnet, dass** die Ivermectinmenge 0,001 bis 10 Gew.-%, vorzugsweise 0,001 bis 5 Gew.-% in Bezug auf das Gesamtgewicht der Zusammensetzung ausmacht.

6. Zusammensetzung nach einem der Ansprüche 1 bis 5, **dadurch gekennzeichnet, dass** die Fettphase weiterhin eine Fettphase, die den Wirkstoff nicht löst und in der die Zusammensetzung der Familie der Avermectine eine Löslichkeit von 1 Gew. -% oder weniger in Bezug auf das Gesamtgewicht der nichtlösenden Fettphase ausmacht, umfasst.

7. Zusammensetzung nach Anspruch 6, **dadurch gekennzeichnet, dass** die Fettphase, die den Wirkstoff nicht löst, aus Silikonölen, Mineralölen, Stearylalkohol, Cetylalkohol, Wachsen, Buttern und ihren Mischungen ausgewählt ist.

8. Zusammensetzung nach einem der Ansprüche 1 bis 7, **dadurch gekennzeichnet, dass** die wässrige Phase in einer Menge zwischen 30 und 95 Gew. -% in Bezug auf das Gesamtgewicht der Zusammensetzung vorliegt.

9. Zusammensetzung nach einem der Ansprüche 1 bis 8, **dadurch gekennzeichnet, dass** sie Folgendes umfasst:
0,01 bis 25% Fettphase, die den Wirkstoff löst
0 bis 20% Fettphase, die den Wirkstoff nicht löst
1 bis 8% Emulgator
0 bis 5% Geliermittel
0,001 bis 5% Ivermectin
50 bis 75% wässrige Phase.

10. Zusammensetzung nach einem der Ansprüche 1 bis 9, **dadurch gekennzeichnet, dass** sie Folgendes umfasst:
5 bis 20% Fettphase, die den Wirkstoff löst
0 bis 10% Fettphase, die den Wirkstoff nicht löst
2 bis 5% Emulgator
0 bis 3% Geliermittel
0,001 bis 2% Ivermectin
55 bis 70% wässrige Phase.

11. Verfahren zur Herstellung einer Zusammensetzung nach einem der Ansprüche 1 bis 10, das die folgenden Schritte umfasst:
a) Mischen des Ivermectins mit mindestens einem öligen Lösungsmittel, bis das Ivermectin gelöst ist, um die Fettphase zu bilden;
b) Mischen der Bestandteile der wässrigen Phase, bis die Mischung homogen ist;
c) Einarbeiten der Fettphase in die wässrige Phase, um eine Emulsion zu bilden.

12. Verfahren nach Anspruch 11, **dadurch gekennzeichnet, dass** es zwischen den Schritten a) und b) einen Schritt des Mischens der Fettphase mit mindestens einer lipophilen Verbindung, die den Wirkstoff nicht löst, umfasst.

13. Verwendung einer Zusammensetzung nach einem der Ansprüche 1 bis 10 zur Herstellung eines pharmazeutischen Präparats zur Behandlung von Rosacea, Acne vulgaris, seborrhoischer Dermatitis, perioraler Dermatitis, akneförmigen Eruptionen, vorübergehender akantolytischer Dermatitis und Acne miliaris necrotica bestimmt ist.

14. Verwendung nach Anspruch 13, **dadurch gekennzeichnet, dass** das pharmazeutische Präparat zur Behandlung von Rosacea bestimmt ist.

## Claims

1. Pharmaceutical composition comprising at least one fatty phase, at least one aqueous phase and at least one compound of the avermectin family, **characterized in that** the fatty phase comprises at least one oily solvent chosen from diisopropyl adipate, PPG 15 stearyl ether, octyl dodecanol, C₁₂-C₁₅ alkyl benzoate and mixtures thereof in which the compound of the avermectin family is solubilized.

2. Pharmaceutical composition according to Claim 1, **characterized in that** the compound of the avermectin family is chosen from ivermectin, invermectin, avermectin, abamectin, doramectin, eprinomectin and selamectin.

3. Pharmaceutical composition according to either of Claims 1 and 2, **characterized in that** the compound of the avermectin family is ivermectin.

4. Composition according to one of Claims 1 to 3, **characterized in that** it is in the form of an oil-in-water emulsion.

5. Composition according to one of the preceding claims, **characterized in that** the amount of ivermectin represents from 0.001% to 10%, and preferably from 0.001% to 5% by weight, relative to the total weight of the composition.

6. Composition according to one of Claims 1 to 5, **characterized in that** the fatty phase also comprises a fatty phase which is not a solvent for the active agent in which the compound of the avermectin family has a solubility of less than or equal to 1% by weight relative to the total weight of the non-solvent fatty phase.

7. Composition according to Claim 6, **characterized in that** the fatty phase which is not a solvent for the active agent is chosen from silicone oils, mineral oils, stearyl alcohol, cetyl alcohol, waxes, butters and mixtures thereof,

8. Composition according to one of Claims 1 to 7, **characterized in that** the aqueous phase is present in an amount of between 30% and 95% by weight, relative to the total weight of the composition.

9. Composition according to one of Claims 1 to 8, **characterized in that** it comprises:
0.01% to 25% of fatty phase which is a solvent for the active agent
0 to 20% of fatty phase which is not a solvent for the active agent
1% to 8% of emulsifier
0 to 5% of a gelling agent
0.001% to 5% of ivermectin
50% to 75% of aqueous phase.

10. Composition according to one of Claims 1 to 9, **characterized in that** it comprises:
5% to 20% of fatty phase which is a solvent for the active agent
0 to 10% of fatty phase which is not a solvent for the active agent
2% to 5% of emulsifier
0 to 3% of a gelling agent
0.001% to 2% of ivermectin
55% to 70% of aqueous phase.

11. Method for preparing the composition according to one of Claims 1 to 10, comprising the following steps:
a) mixing ivermectin with at least one oily solvent until the ivermectin is solubilized, in order to form the fatty phase;
b) mixing the constituents of the aqueous phase to homogeneity;
c) incorporating the fatty phase into the aqueous phase so as to form an emulsion.

12. Method according to Claim 11, **characterized in that** it comprises, between steps a) and b), a step of mixing the fatty phase with at least one lipophilic compound which is not a solvent for the active agent.

13. Use of the composition according to one of Claims 1 to 10, in the manufacture of a pharmaceutical preparation for use in treating rosacea, acne vulgaris, seborrhoeic dermatitis, perioral dermatitis, acneiform eruptions, transient acantholytic dermatitis and acne *miliaris necrotica.*

14. Use according to Claim 13, **characterized in that** the pharmaceutical preparation is for use in treating rosacea.
